# EUROPEAN PATENT APPLICATION

(11) **EP 1 854 432 A1**
(43) Date of publication of application: **14.11.2007**
(21) Application number: 07107868.7
(22) Date of filing: 09.05.2007
(51) Int. Cl.: A61F 2/38

(54) **Knee prosthesis**

(30) Priority: 09.05.2006 GB 0609058
(71) Applicant: Finsbury (Development) Limited, Leatherhead, Surrey KT22 0BA (GB)
(72) Inventor: Tuke, Michael Antony, Langton, Surrey GU1 3TF (GB)
(74) Representative: Smaggasgale, Gillian Helen

(57) **Abstract**

A tibial plate (1) for an endoprosthetic knee is provided, the plate having a proximal face (2), a distal face (3) and at least one distal posterior reinforcement portion (13) projecting from a posterior region of the distal face, said reinforcement portion having an abutment face (14) bevelled with respect to the distal surface of the tibial plate. A distal anterior posterior reinforcement portion (8) projecting from an anterior region of the distal face may also be included.

## Description

The present invention relates to knee prosthesis and, in particular, to a tibial plate for use in endoprosthetic meniscal knees.

Conventionally, two principal types of endoprosthetic knee are known. The first type, the so-called total knee, comprises a tibial plate and a femoral component with an intervening meniscal component, each component having medial and lateral sides. Typically the tibial plate and femoral compartment are made from a suitable metal or metal alloy, such as an alloy of cobalt and chromium, whereas the meniscal components are made from a synthetic plastics material, for example ultra high molecular weight polyethylene. In most designs the meniscal component is fixed to the tibial plate. In other designs the meniscal component is free to float to a certain extent with respect to the tibial plate in order that the prosthesis shall mimic better the natural movement of the knee. The total knee is designed to replace all of the articulating surfaces of the knee.

The second type of endoprosthetic knee, the so-called unicompartmental knee, also has a tibial plate a femoral component and an intervening meniscal component. Again it is usual for the meniscal component to be fixed to the tibia but in some designs a certain floating movement of the meniscal component is provided for. However, in this case, only a medial or, alternatively, a lateral femoro-tibial replacement is provided.

A third type of endoprosthetic knee, referred to herein as the "bi-unicompartmental" type comprises two unicompartmental knee prostheses used, one on each of the lateral and medial sides, to replace all of the articulating surfaces of the femoro-tibial articulation.

In a variation on the total knee prosthesis, it is possible to have a single tibial plate with medial and lateral portions upon which are seated two separate meniscal components, one meniscal component upon each of the respective medial and lateral portions of the tibial plate.

The present invention is applicable to all of the above mentioned types of endoprosthetic knee.

Knee arthroplasty requires, inter alia, resection of the proximal tibia. A prosthetic tibial plate is then anchored to the resected surface, generally by means of one or more anchoring pegs, posts or screws which are cemented or simply inserted into one or more longitudinal cavities formed in the shaft of the tibia, from the proximal resected surface thereof.

Resection of the proximal tibia necessarily weakens the load-bearing capacity of the bone since the bone becomes increasingly sponge-like beneath the surface. To preserve as much as possible of the natural bone, the tibial resection in knee arthroplasty is generally made as a horizontal cut across the tibia, that is to say a cut in a plane which would be horizontal when the patient is standing erect. However, this has the effect of increasing the potential shear load across the anterior portion of the tibia when the prosthesis is fitted because it does not compensate for the natural slope of the proximal tibia, which in the natural knee is angled downwardly from the horizontal towards the posterior of the tibia with a slope of about 5°-10°.

One conventional endoprosthetic knee addresses this problem to a certain extent by requiring the surgeon to make a tibial resection at an angle of between 5 and 10° to the horizontal, thus mimicking the natural knee in this respect.

A further problem associated with known knee prosthesis is that whilst they generally do provide the patient with a level of movement of the knee join which allows the patient to walk, they do not generally provide the full range of motion noted in a normal healthy knee. For a normal knee to achieve full flexion, the articular cartilage actually rolls slightly off the back surface of the tibia. This has not to date been achievable with the knee prosthesis of the prior art.

Whatever the plane of the proximal tibia resection, a flat cut is normally used. This has the disadvantage of relying on the fixation means for all shear loads in both translation and rotation.

It is therefore an object of the present invention to provide an improved tibial plate for endoprosthetic knees.

Accordingly, the present invention provides a tibial plate for an endoprosthetic knee, the plate having a proximal face, a distal face and at least one distal posterior reinforcement portion projecting from a posterior region of the distal face, said reinforcement portion having an abutment face bevelled with respect to the distal surface of the tibial plate.

Preferably, the proximal face is smooth to allow glidable movement of a prosthetic meniscal component thereon.

To make use of a tibial plate according to the invention, the surgeon should therefore make two cuts on the proximal tibia. The first cut is at 5-10° to the horizontal, sloping downwardly towards the posterior region of the tibia. The second cut is made at an angle to the first cut so as to make a chamfered edge at the posterior region of the resected tibia.

An advantage of endoprosthetic knees utilising tibial plates in accordance with the invention is that the torsional loads and translational loads on the tibia are supported by the distal reinforcement portion, thus providing a fitting which is more secure and less likely to loosen than conventional tibial plates. The load-bearing capacity of the tibia is increased compared to those of conventional endoprosthesis.

A further advantage of the present invention is that the posterior projection enables movement which more accurately reflects the natural movement in that the presence of the posterior projection provides the lateral femoral condyle space to roll back when the knee is in deep flexion.

A still further advantage of the present invention is that there may be increased resistance to subsidence of tibial prosthesis into the cut bone due to the increased surface area.

In a preferred arrangement of the present invention, the endoprosthetic knee may additionally include at least one distal anterior reinforcement portion projecting from an anterior region of the distal face, said reinforcement portion having an abutment face bevelled with respect to the distal surfaces of the tibial plate.

Where the distal anterior reinforcement portion is present in the endoprosthetic knee, the surgeon will need to make three cuts. The first and second cuts will be as detailed above and the third will be made at an angle to the first cut so as to make a chamfered edge at the anterior region of the resected tibia.

In a preferred embodiment of the invention, the bevel angle formed between the abutment face of the reinforcement portion and the distal face of the tibial plate is from about 100° to about 130°. The angle of the posterior reinforcement portion to the distal face of the tibial plate may be the same or different to that of the anterior reinforcement portion.

Still more preferably, the tibial plate and the reinforcement portion(s) are of unitary construction.

A tibial plate in accordance with the invention may be designed for use in a unicompartmental endoprosthetic knee. In this case the tibial plate is designed so as to replace the lateral only or alternatively the medial portion only of the natural tibial head. Alternatively, a tibial plate according to the invention may be designed to form part of a total endoprosthetic knee in which it is intended to replace both the lateral and medial portions of the natural tibia.

A tibial plate according to the invention for use in a total endoprosthetic knee may be provided with a posterior cut-out portion for accommodating a patient's posterior cruciate ligament. Optionally, the cut-out portion may be extended towards the anterior region of the plate in order that the patient's anterior cruciate ligament may also be accommodated therein. This form of total knee tibial component is made feasible by the invention which provides, by way of the distal reinforcement portion, a strong bridge on the anterior tibia. This embodiment of the invention may be regarded as a total knee tibial plate in which two unicompartmental tibial plates are joined, allowing the anterior cruciate ligament to be preserved.

It will be acknowledged that the surface of the meniscal component may be of any suitable configuration. For example, it may be flat, convex or concave.

The tibial plate of the present invention may be connected to the tibia by any suitable means. In one arrangement, anchoring means will be provided on the distal face or anchoring the plate to the proximal end of a resected tibia.

The present invention will now be described, by way of example, and so that the invention may be clearly understood and readily carried into effect. The description will be made with particular reference to the accompanying drawings in which:
- Figure 1(a): shows a top plan view, partially in outline of a tibial plate in accordance with the invention and a corresponding meniscal component which together form part of a unicompartmental endoprosthetic knee;
- Figure 1(b): shows a cross-section on line A-A of Figure 1 (a) together with a side elevational view of an appropriately resected tibia;
- Figure 2(a): shows a top plan view, partially in outline, of two mirror image tibial plates in accordance with the invention and corresponding meniscal components which together form part of a bi-unicompartmental endoprosthetic knee;
- Figure 2(b): shows a cross-section on line B-B of Figure 2(a) together with a side elevational view of an appropriately resected tibia;
- Figure 3(a): shows a top plan view, partially in outline, of a tibial plate in accordance with the invention and a corresponding meniscal component which together form part of a first total endoprosthetic knee;
- Figure 3(b): shows a cross-section on line C-C of Figure 3(a) together with a side elevational view of an appropriately resected tibia;
- Figure 4(a): shows a top plan view, partially in outline, of a tibial plate in accordance with the invention and two corresponding mirror-image meniscal components which together form part of a second total endoprosthetic knee;
- Figure 4(b): shows a cross-section on line D-D of Figure 4(a) together with a side elevational view of an appropriately resected tibia;
- Figure 5(a): shows a top plan view, partially in outline, of a tibial plate in accordance with the invention and two corresponding mirror-image meniscal components which together form part of a third total endoprosthetic knee.
- Figure 5(b): shows a cross-section on line E-E of Figure 5(a) together with a side elevational view of an appropriately resected tibia.

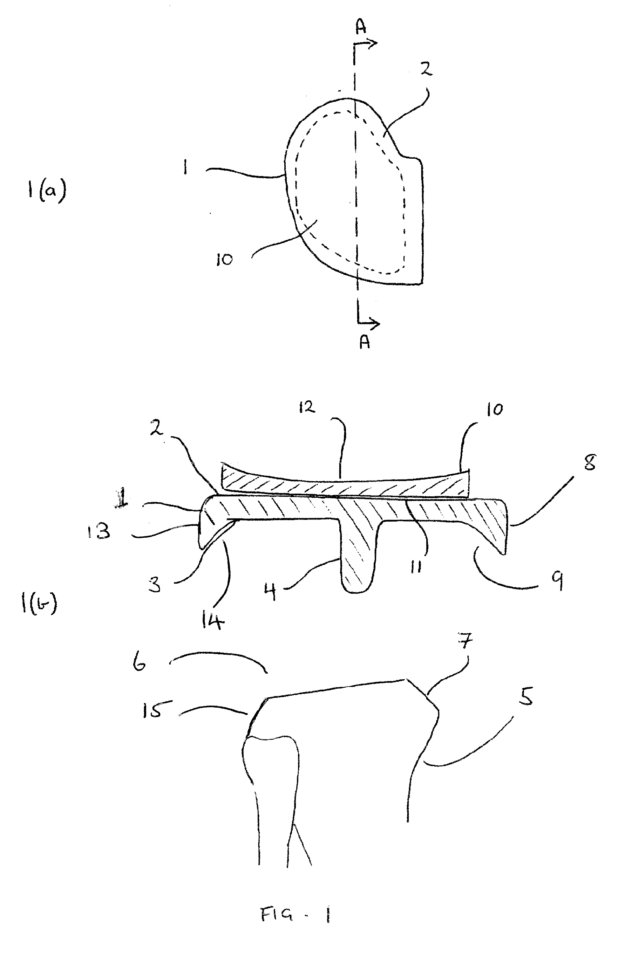

Figures 1 to 5 are not drawn to scale. The invention is illustrated for an arrangement having both posterior and anterior reinforcement portions. However, it will be understood that the anterior reinforcement portion may be omitted.

Referring to Figure 1 there is shown a tibial plate 1 having a smooth proximal face 2 and a distal face 3. Tibial plate 1 comprises a longitudinal pin 4 extending from the distal face 3 of tibial plate 1, pin 4 being adapted for insertion into a longitudinal cavity (not shown) drilled in the patient's tibia 5 to anchor the tibial plate to the bone. Optionally, ancillary anchoring means may also be used. These may comprise one or more further pegs which may be ribbed for extra surface area contact with bone or cement. In addition, distal face 3 may be dimpled to provide better contact with the bone surface. Such methods and means for anchoring the tibial plate to the bone are known and are well understood by those skilled in the art. Tibial plate 1 is preferably made from an alloy of cobalt and chromium. The dimensions of tibial plate 1 may vary for different patients and/or for different indications. Particularly, the thickness of tibial plate 1 is normally from about 3mm to about 5mm.

Tibia 5 has an angled and posteriorly sloping resected face 6, a chamfered anterior edge portion 7 and a chamfered posterior edge portion 15 formed by the surgeon cutting an anterior part of the tibia 5 at an angle of approximately 30°-45° to the horizontal.

Tibial plate 1 is further provided with distal reinforcement portions 8 and 13 which project from anterior and posterior regions respectively of distal surface 3. Distal reinforcement portions 8 and 13 have respective abutment surfaces 9 and 14 bevelled with respect to distal surface 3 of tibial plate 1.

When tibial plate 1 is seated on tibia 5, abutment surface 9 is arranged to seat on the resected tibia at chamfered edge portion 7, thereby providing additional surface area contact between tibial plate 1 and tibia 5 in the anterior region of tibia 5. In addition, abutment surface 14 is arranged to seat on the resected tibia at chamfered edge portion 15 thereby providing additional surface area contact in the posterior region of the tibia.

Meniscal component 10 is seated on tibial plate 1 and is shown only in outline in Figure 1(a). Meniscal component 10 may have a substantially flat distal face 11 for glidable seating on proximal face 2 of tibial plate 1. Meniscal component 10 also has a dished proximal face 12 for receiving a femoral component (not shown). Meniscal component 10 may be constructed from ultra high molecular weight polyethylene. Alternatively, a metal component 10, for example of cobalt chromium alloy, may be used. The dimensions of the meniscal component may vary for different patients and/or indications. In particular, the thickness of the meniscal component is normally between about 6mm and 15mm at the thinnest portion of the meniscal component.

Endoprosthetic knees utilising tibial plates according to the invention maybe manufactured to fit various bone sizes. The combined thickness of the tibial plate and meniscal component together with the femoral component for use therewith may be chosen to match a particular flexion/extension gap which the surgeon has cut. Normally, femoral components which are commercially available are manufactured with a standard thickness of about 9mm. The combined thickness of the tibial plate and meniscal component is preferably between about 7mm and about 17mm.

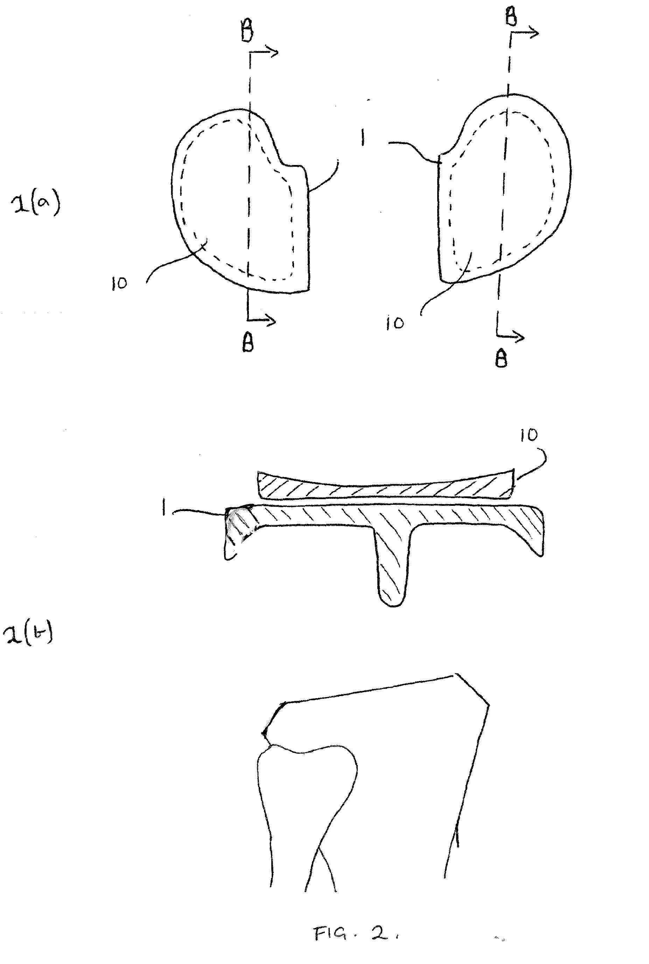

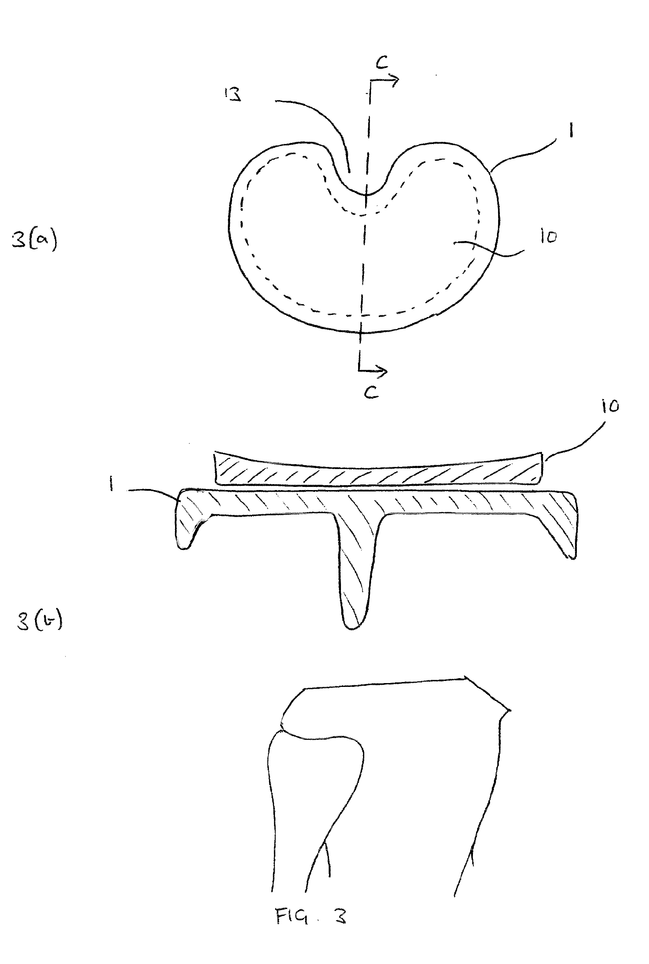

Referring to Figure 2, there is shown a pair of mirror image tibial plates 1 according to the invention and a corresponding pair of meniscal components 10 for use in a bi-unicompartmental endoprosthetic knee.

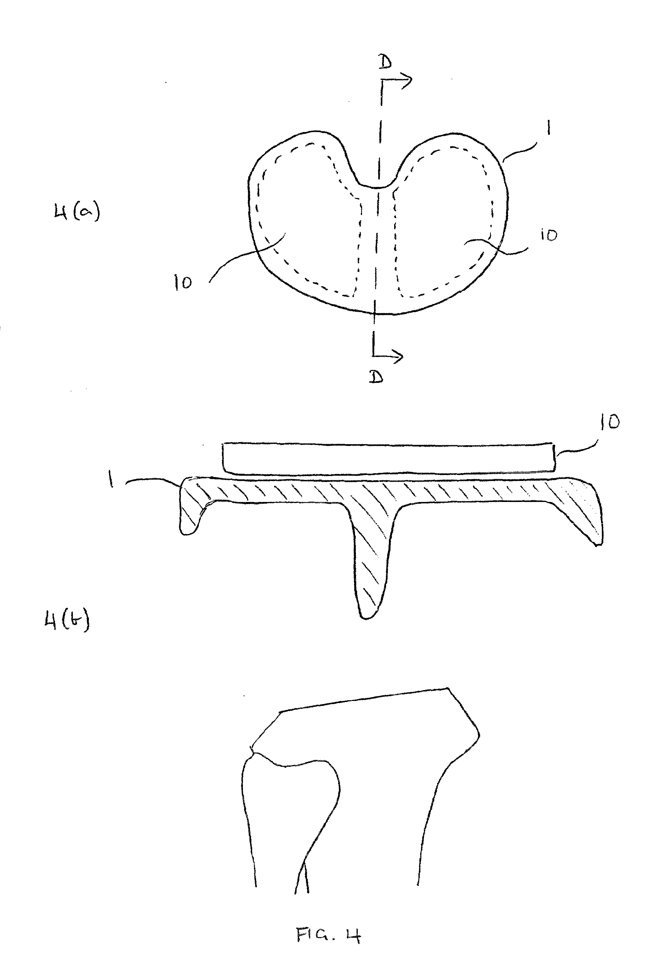

Referring to Figure 3, there is shown a tibial plate 1 in accordance with the invention and a corresponding meniscal component 10 for use in a first total, endoprosthetic knee. Cut-out portion 13 is provided to accommodate, in use, the patient's posterior cruciate ligament.

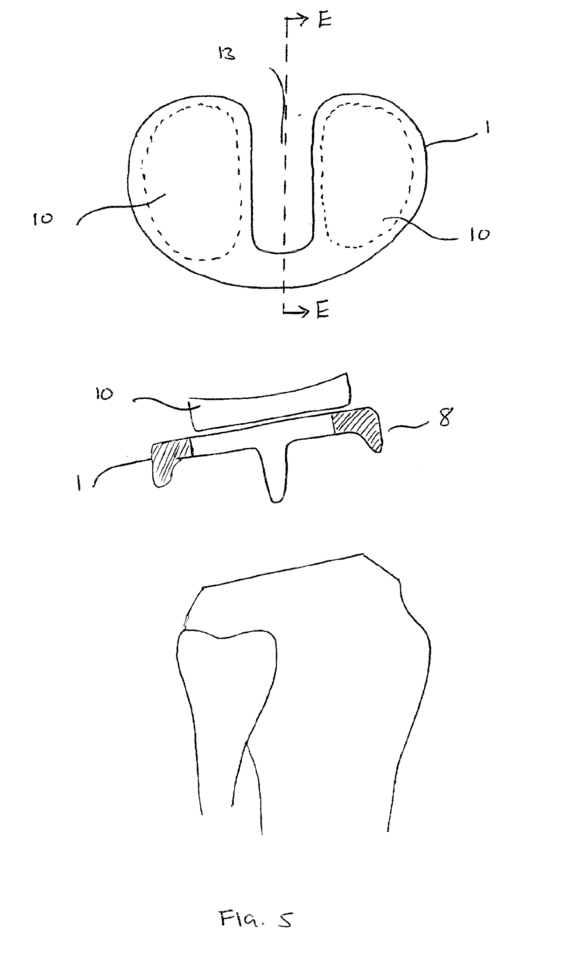

Referring to Figure 4, there is shown a tibial plate 1 in accordance with the invention and two corresponding mirror image meniscal components 10 for use in a second total endoprosthetic knee.

Referring to Figure 5, there is shown a tibial plate 1 in accordance with the invention and two corresponding mirror image meniscal components 10 for use in a third total endoprosthetic knee. Tibial plate 1 is provided with a posterior cut-out portion 13 which extends towards the anterior region of tibial plate 1 to accommodate, in use, the patient's posterior and anterior cruciate ligaments. The additional strength provided to the plate by distal reinforcement portion 8 permits a cut-out portion 13 of this magnitude.

## Claims

**1.** A tibial plate for an endoprosthetic knee, the plate having a proximal face, a distal face and at least one distal posterior reinforcement portion projecting from a posterior region of the distal face, said reinforcement portion having an abutment face bevelled with respect to the distal surface of the tibial plate.

**2.** A tibial plate according to Claim 1, wherein the bevel angle formed between the abutment face of the posterior member and the distal face of the tibial plate is from about 100 to about 130°.

**4.** A tibial plate according to Claim 1 or 2, wherein the tibial plate and the reinforcement portion are of unitary construction.

**5.** A tibial plate according to any one of Claims 1 to 4, wherein the abutment face of the posterior projecting portion is the posterior-most distal region of the plate.

**6.** A tibial plate according to any one of Claims 1 to 5, wherein the projecting portion extends along substantially the full width of the plate.

**7.** A tibial plate according to any one of Claims 1 to 6, additionally including at least one distal anterior reinforcement portion projecting from an anterior region of the distal face, said reinforcement portion having an abutment face bevelled with respect to the distal surface of the tibial plate.

**8.** A tibial plate according to Claim 7, wherein the bevel angle formed between the abutment face of the anterior member and the distal face of the tibial plate is from about 100 to about 130°.

**9.** A tibial plate according to Claim 7 or 8, wherein the tibial plate and the reinforcement portion are of unitary construction.

**10.** A tibial plate according to any one of Claims 7 to 9, wherein the abutment face of the anterior projecting portion is the anterior-most distal region of the plate.

**11.** A tibial plate according to any one of Claims 7 to 10, wherein the anterior projecting portion extends along substantially the full width of the plate

**12.** A tibial plate according to any one of Claims 1 to 11 which is adapted for use in a unicompartmental endoprosthetic knee and is shaped to replace the lateral portion of, or alternatively the medial portion of, the natural proximal tibia.

**13.** A tibial plate according to any one of Claims 1 to 11 which is adapted for use in a total endoprosthetic knee and is shaped to replace both the lateral and medial portions of the natural proximal tibia.

**14.** A tibial plate according to any one of Claims 1 to 13, wherein the plate is provided with a posterior cut-out portion for accommodating, in use, a patient's posterior cruciate ligament.

**15.** A tibial plate according to Claim 14, wherein the cut-out portion extends sufficiently towards the anterior region of the tibial plate such that, in use, the patient's posterior and anterior cruciate ligaments may be accommodated within the cut-out portion.

**16.** A set of tibial plates according to any one of Claims 1 to 15, which is adapted for use in a bi-unicompartmental endoprosthetic knee, which set comprises a first tibial plate shaped to replace the lateral portion of the natural proximal tibia and a second tibial plate shaped to replace the medial portion of the natural proximal tibia head.

**17.** A unicompartmental knee prosthesis comprising a tibial plate according to any one of Claims 1 to 15.

**18.** A bi-unicompartmental knee prosthesis comprising two tibial plates according to any one of Claims 1 to 15.

**19.** A total knee prosthesis comprising a tibial plate according to any one of Claims 1 to 15.
